Europäisches Patentamt

European Patent Office

Office européen des brevets

(11) Veröffentlichungsnummer: **0 180 720**
**B1**

(12) EUROPÄISCHE PATENTSCHRIFT

(45) Veröffentlichungstag der Patentschrift: **13.06.90**

(51) Int. Cl.⁵: **A 61 M 1/34**, G 01 N 33/92

(21) Anmeldenummer: **85109470.6**

(22) Anmeldetag: **08.09.82**

(80) Veröffentlichungsnummer der früheren Anmeldung nach Art. 76 EPÜ: **0 074 610**

(54) **Vorrichtung zur selektiven extrakorporalen Präzipation von Low Density Lipoprotenen aus Vollserum oder Plasma.**

(30) Priorität: **10.09.81 DE 3135814**
**13.05.82 DE 3217925**

(43) Veröffentlichungstag der Anmeldung:
**14.05.86 Patentblatt 86/20**

(45) Bekanntmachung des Hinweises auf die Patenterteilung:
**13.06.90 Patentblatt 90/24**

(84) Benannte Vertragsstaaten:
**AT BE CH DE FR GB IT LI LU NL SE**

(56) Entgegenhaltungen:
**EP-A-0 044 694**
**DE-A-2 027 087**
**DE-B-2 345 994**
**US-A-4 103 685**
**US-A-4 223 672**

**PATENTS ABSTRACTS OF JAPAN, Band 5, nr.
89, 1. Juni 1981; & JP-A-56 33 017 (TERUMO
K.K.) 03-04-1981**

(73) Patentinhaber: **B. Braun-SSC AG**
**Gerliswilstrasse 74**
**CH-6020 Emmenbrücke (CH)**

(72) Erfinder: **Seidel, Dietrich, Prof.**
**Dahlmannstrasse 23**
**D-3400 Göttingen (DE)**
Erfinder: **Wieland, Heinrich, Dr.**
**Wiesenweg 2**
**D-3401 Waake (DE)**
Erfinder: **Rath, Dieter**
**Franz-Gleim-Strasse 67**
**D-3508 Melsungen (DE)**
Erfinder: **Rosskopf, Gerhard, Dr.**
**Heiligenbergstrasse 29**
**D-3501 Fuldabrück-Dörnhagen (DE)**

(74) Vertreter: **von Kreisler, Alek, Dipl.-Chem. et al**
**Deichmannhaus am Hauptbahnhof**
**D-5000 Köln 1 (DE)**

## Beschreibung

Die Erfindung betrifft eine Vorrichtung zur extra-korporalen Präzipitation von Blutbestandteilen, mit einem Behälter, dem Blut oder Blutbestandteile wie z.B. Plasma eines Patienten über eine erste Pumpe zugeführt werden, und einem dem Behälter nachgeschalteten Filter dessen Filtrat-Auslaß mit einer zum Patienten zurückführenden Leitung verbunden ist.

Die Fortschritte auf dem Gebiet der Analytik des Lipoproteinsystems der letzten Jahre haben ergeben, daß die in älteren epidemiologischen Daten erhobenen engen Korrelationen zwischen Plasmacholesterinkonzentrationen und dem Risiko einer frühzeitigen Atherosklerose, speziell der Koronarsklerose, im wesentlichen durch cholesterinreiche β-Lipoproteine gegeben sind. Im Blut des Menschen finden sich normalerweise ca. 70—80% des gesamten Cholesterins an die low density bzw. β-Lipoproteine gebunden, der Rest verteilt sich auf andere Lipoproteinfraktionen (im wesentlichen VLDL, HDL und Chylomikronen) (LDL = Low Density Lipoproteine; VLDL = Very Low Density Lipoproteine; HDL = High Density Lipoproteine). In Krankheitsprozessen, die mit einem gestörten Fettstoffwechsel bzw. erhöhten Plasmalipidkonzentrationen einhergehen und die zur frühzeitigen Atherosklerose führen, kann sich der prozentuale Anteil des LDL- oder β-Cholesterins (LDL = β-Lipoprotein) am Gesamtcholesterin sogar noch weit erhöhen. Das heisst, eine Hypercholesterinämie ist in der Regel durch eine Hyper-β-Lipoproteinämie hervorgerufen. In der Vergangenheit hat es daher auch nicht an Versuchen gefehlt

1. die low density Lipoproteine selektiv zu messen und

2. solche, möglichst selektiv aus der Zirkulation zu eliminieren.

Die US—A—4 103 685 offenbart eine Vorrichtung zur Entfernung von β- und pre-β-Lipoproteinen aus Blut, die aus einem Aufnahmebehälter für das Blut, einer Anordnung zur Entfernung des behandelten Bluts und einer dazwischen angebrachten Filteranordnung besteht.

Die US—A—4 223 672 beansprucht eine Vorrichtung zur extrakorporalen Behandlung von Krankheiten, bestehend aus einer Anordnung zum Entnehmen von Vollblut von Patienten, einer Anordnung zum Abtrennen des Plasmas vom Vollblut, einer Anordnung zur Behandlung des Plasmas mit einer Kammer zum Auffangen des Plasmas und einem Behälter innerhalb der Kammer mit einem darauf fixierten Immunoadsorbens, der mit einer immunologisch aktiven Komponente des Plasmas reagiert und einer Anordnung zum Rekombinieren des von der immunologisch aktiven Komponente befreiten Plasmas mit dem Rest des Vollblutes und zum Rückführen des rekombinierten Vollbluts zum Patienten.

Der Erfindung liegt die Aufgabe zugrunde, eine Vorrichtung dieser Art so zu gestalten, daß sie ohne aufwendige Durchflußmengenregelung in der Betriebsphase kontinuierlich arbeitet, um den Patienten so wenig wie möglich zu belasten und um aufwendige und somit störanfällige Regelsysteme zu vermeiden.

Diese Aufgabe ist erfindungsgemäß gelöst durch eine Vorrichtung zur extra-korporalen Präzipitation von Blutbestandteilen, mit einem Behälter, dem Blut oder Blutbestandteile eines Patienten über eine erste Pumpe zugeführt werden, und einem dem Behälter nachgeschalteten Filter dessen Filtrat-Auslaß mit einer zum Patienten zurückführenden Leitung verbunden ist, dadurch gekennzeichnet, daß das Filter (21) einen zweiten Auslaß für das Präzipitat aufweist, der mit einer in den Behälter (16) führenden Rücklaufleitung (221) verbunden ist, daß der Behälter (16) über eine zweite Pumpe (13) mit dem Auslaß eines ein Behandlungsmittel enthaltenden ersten Gefäßes (15) verbunden ist, und daß der Filtrat-Auslaß (211) des Filters (21) an eine dritte Pumpe (23) angeschlossen ist, deren Förderleistung im wesentlichen der Summe der Förderleistungen der ersten und der zweiten Pumpe entspricht.

Hierbei bildet der Behälter zusammen mit dem Filter und der Rücklaufleitung einen geschlossenen Kreis, in dem Flüssigkeit zirkuliert. In diesen Kreis gelangt externe Flüssigkeit, über die in den Behälter hinein Blut oder Blutbestandteile fördernde erste Pumpe und über die zweite Pumpe, die das Behandlungsmittel, im vorliegenden Fall Heparin, in den Behälter pumpt. Diesem geschlossenen Kreislauf wird an dem Filtrat-Auslaß des Filters Flüssigkeit durch die dritte Pumpe entnommen. Dadurch, daß die Förderleistung der dritten Pumpe auf die Summen der Förderleistungen der ersten und der zweiten Pumpe abgestimmt ist, wird das Flüssigkeitsgleichgewicht im Kreislauf im wesentlichen aufrechterhalten, so daß der Flüssigkeitsstand im Behälter stets annähernd gleichbleibt. Daher ist ein häufiges Zu- und Abschalten der ersten Pumpe nicht erforderlich, so daß dem Patienten gleichmäßig Blut entnommen werden kann. Dabei ist zu berücksichtigen, daß es sich bei den drei genannten Pumpen um volumetrische Pumpen handelt, deren Fördervolumen der Antriebsdrehzahl proportional ist. Um zu verhindern, daß die zu pumpende Flüssigkeit kontaminiert wird, werden zweckmäßigerweise Schlauchpumpen benutzt.

Die Förderleistung der ersten Pumpe wird maßgeblich vom Verhalten des Körpers des Patienten bestimmt, da verhindert werden muß, daß dem Patienten zuviel bzw. zu schnell Blut entnommen wird. Daher ist das System zweckmäßigerweise so angelegt, daß die Förderleistung der ersten Pumpe verändert werden kann, ohne daß das Umlaufsystem aus dem Gleichgewicht gebracht wird. Hier- zu ist in vorteilhafter Weiterbildung der Erfindung vorgesehen, daß die Förderleistung der ersten Pumpe in Abhängigkeit von den Verhältnissen der Blutentnahme am Patienten veränderbar ist und daß die Antriebe der ersten, der zweiten und der dritten Pumpe miteinander derart gekoppelt sind, daß das Verhältnis der drei Förderleistungen konstant bleibt.

Von Vorteil ist hierbei, daß das System mit zeitlich varierenden Fördermengen betrieben werden kann, ohne daß das Flüssigkeitsgleichgewicht verloren geht. Die Pumpen sind über ihre Antriebe entweder mechanisch oder elektrisch miteinander gekoppelt, wobei die erste Pumpe die Führung übernimmt und sich die Drehzahlen der beiden anderen Pumpen selbsttätig entsprechend anpassen.

Trotz der gegenseitigen Abstimmung der Förderleistungen der drei Pumpen kann es vorkommen, daß bei längerem Betrieb der Vorrichtung, der Flüssigkeitsstand im Behälter zunehmend ansteigt. Um diesem Ansteigen eine Grenze zu setzen, weist der Behälter mindestens einen Füllstandsdetektor auf, der bei einem oberen Füllstand die erste Pumpe auf eine geringere Förderleistung umstellt oder abstellt.

Um andererseits ein zu starkes Absinken des Flüssigkeitsstandes in dem Behälter zu vermeiden, wird die dritte Pumpe von dem Füllstandsdetektor auf eine geringere Pumpenleistung umgestellt oder abgestellt, wenn ein unterer Füllstand erreicht ist. Diese Maßnahmen der Begrenzung des Füllstandes auf den Bereich zwischen dem unteren und dem oberen Wert dient dazu, den Einfluß sich ständig summierender Fehlabweichungen im Flüssigkeitsgleichgewicht des Kreislaufs zu begrenzen.

Da sich das Filter nach einiger Zeit zusetzt, ist in vorteilhafter Weiterbildung der Erfindung eine Spülphase vorgesehen. Die Vorrichtung weist eine den Strömungswiderstand des Filters messende Einrichtung auf, die bei einem oberen Strömungswiderstand die zweite Pumpe einschaltet, und aus einem ein Lösungsmittel enthaltenden zweiten Gefäß Lösungsmittel in den Behälter fördert und eine Verbindung zwischen dem Auslaß und einem Ablauf herstellt. Auf diese Weise wird der Flüssigkeitskreislauf hinter dem Filter unterbrochen bzw. abgesperrt und sowohl der Behälter als auch das Filter von der Spüllösung durchströmt, die anschließend zusammen mit den Filterrückständen dem Ablauf zugeführt wird.

Im folgenden wird ein Ausführungsbeispiel der erfindungsgemäßen Vorrichtung zur Durchführung einer Plasmapherese mit Präzipitation der low-density-Lipoproteine unter Bezugnahme auf die Zeichnungen näher erläutert.

Es zeigen:

Figur 1 eine schematische Darstellung des Systems, wobei diejenigen Leitungen, die im Betriebszustand durchflossen sind, mit größerer Strichstärke gezeichnet sind, und

Figur 2 das System der Figur 1, wobei diejenigen Leitungen, die beim Spülen des Filters durchflossen sind, mit dickerer Strichstärke gezeichnet sind.

In den Zeichnungen ist oberhalb der gestrichelten Linie das patientenseitige Blutentnahme und -Rückführungssystem dargestellt, das bekannt ist. Über den Patientenanschluß 1 wird dem Patienten unter ständiger Überwachung durch ein Druckmeßgerät 3 Blut entnommen. Dieses Blut wird über eine Pumpe 4 einem Plasmafilter 6 zugeführt, nachdem aus einem Gefäß 5 Antikoagulans zugemischt wurde. Aus dem Filter 6 fließt das um eine gewisse Menge Blutplasma reduzierte Blut durch einen Luftfänger 7 und ein Magnetventil 9 zum Rücklaufanschluß 2 des Patienten. Der Rücklaufdruck wird von einer Druckmeßeinrichtung 8 überwacht.

Das abgezweigte Blutplasma verläßt das Filter 6 durch dessen Filtrat-Ausgang 61. Bei dem Filter 6 handelt es sich z.B. um ein Kapillarfilter, das von dem Blut durchströmt wird, wobei die feineren Blutbestandteile durch die Membranwände hindurch seitlich entweichen, während die gröberen Blutbestandteile zum Luftfänger 7 weitergeleitet werden.

Aus dem Plasmafilter 6 wird mittels einer Pumpe 11 eine vorwählbare Menge des Blutplasma abgezogen. Bei der hier als "erste Pumpe" bezeichneten Pumpe 11 handelt es sich, ebenso wie bei den übrigen in dem System verwendeten Pumpen, um eine Schlauchpumpe bzw. peristaltische Pumpe, die selbstansaugend ist und volumetrisch eine der Antriebsdrehzahl proportionale Menge an Plasma fördert. Zwischen dem Plasmafilter 6 und der ersten Pumpe 11 befindet sich ein Blutleckdetektor 10 sowie ein Druckmeßgerät 12. Der Ausgang der ersten Pumpe 11 ist mit einem Einlaß eines Behälters 16 verbunden.

Ein anderer Einlaß des Behälters 16 ist an den Auslaß einer zweiten Pumpe 13 angeschlossen, deren Einlaß über ein Ventil 14 mit einem Gefäß 15, das eine Acetatpufferlösung mit Heparin enthält, sowie über ein weiteres Ventil 30 mit einem Gefäß 29, das eine Spüllösung enthält, verbindbar ist. Die Einlässe befinden sich am oberen Ende des Behälters 16. Der Behälter 16 ist ferner mit einem Füllstandsdetektor 17 ausgestattet, der bei Erreichen eines bestimmten Füllstandes anspricht.

Der am unteren Ende des Behälters 16 vorgesehene Auslaß ist mit einer weiteren Pumpe 18 verbunden, der ein Ventil 31 parallel geschaltet ist. Der Auslaß der Pumpe 18 ist mit dem Filter 21 verbunden, dessen Auslaß über ein Ventil 22 und eine Rücklaufleitung 221 an einen weiteren Einlaß des Behälters 16 angeschlossen ist. Die Drücke am Einlaß und am Auslaß des Filters 21 werden von Druckmessern 19 und 20 überwacht. Der Filtrat-Auslaß 211 des Filters 21 ist über die dritte Pumpe 23 und ein Ventil 25 mit dem Einlaß eines Dialylators 26 verbunden, welcher spüllösungsseitig von einer Dialysespüllösung aus einem Dialysegerät 27 durchströmt wird. In dem Dialysator 26 werden der Anteil Acetatpuffer und das verbliebene Heparin aus dem Behältnis 15 eliminiert, während das Plasma über eine Pumpe 28 dem Luftfänger 7 und somit dem extrakorporalen Blutkreislauf zufließt. Die Pumpe 28 läuft mit gleicher Geschwindigkeit wie die Pumpe 11, damit die Flüssigkeitsbilanz des zu behandelnden Patienten gewährt bleibt. Durch die Dialysierspülflüssigkeit aus dem Dialysegerät 27 erfolgt im Filter 26 gleichzeitig eine Anhebung des pH-Wertes und der Temperatur des rückfließenden Plasmas auf die Körperwerte. Statt eines Dialysegerätes kann auch über einen offenen Kreislauf dem Dialysator 20 eine Flüssigkeit zugeführt werden.

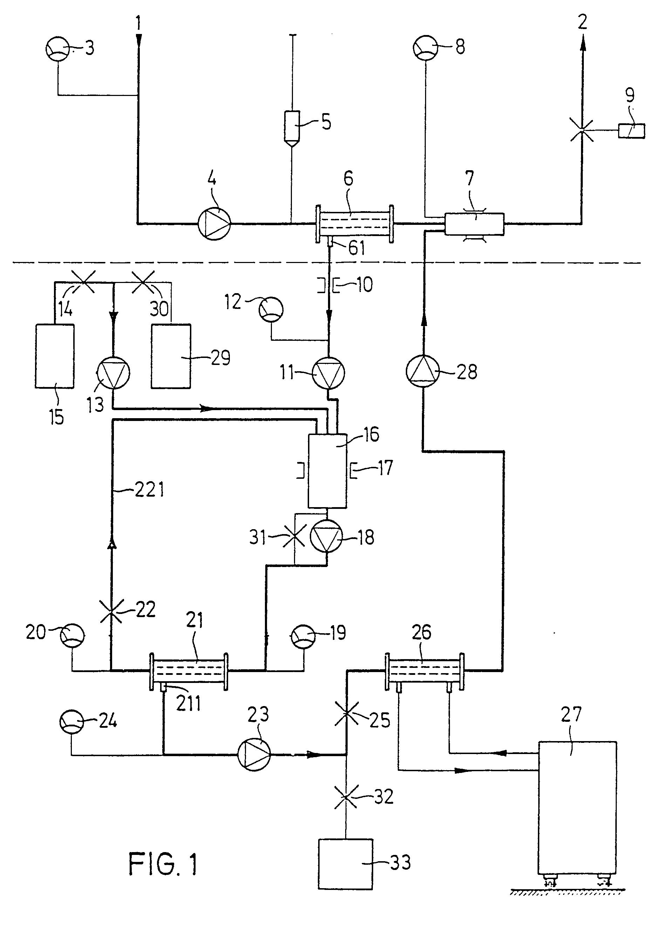

Bei der in Figur 1 dargestellten Betriebsart wird zunächst durch die Pumpe 11 Blutplasma in den Behälter 16 gepumpt, während gleichzeitig über die Pumpe 13 Acetatpufferlösung mit Heparin über das geöffnete Ventil 14 zugeführt wird. In diesem Zustand ist die dritte Pumpe 23 zunächst abgeschaltet. Nach Erreichen des vorgesehenen Füllstandes im Behälter 16 spricht der Füllstandsdetektor 17 an und schaltet die Pumpe 23 ein. Die Pumpe 23 läuft jetzt mit einer Geschwindigkeit, die der Summe der Geschwindigkeiten der Pumpen 11 und 13 entspricht. Die dritte Pumpe 23 fördert somit Plasma aus dem Filter 21 ab, während das Präzipitat wegen der gewählten Porengröße im Filter 21 zurückgehalten wird. Die weitere Pumpe 18, die den Flüssigkeitsumlauf in dem Kreislauf aufrecht erhält, hat eine relativ große Förderleistung, so daß das die low-density-Lipoproteine enthaltende Präzipitat über die Rücklaufleitung 221 in den Behälter 16 zurückgespült wird.

Das von den low-density-Lipoproteinen befreite Plasma wird über die dritte Pumpe 23 und das geöffnete Ventil 25 dem Dialysator 26 zugeführt.

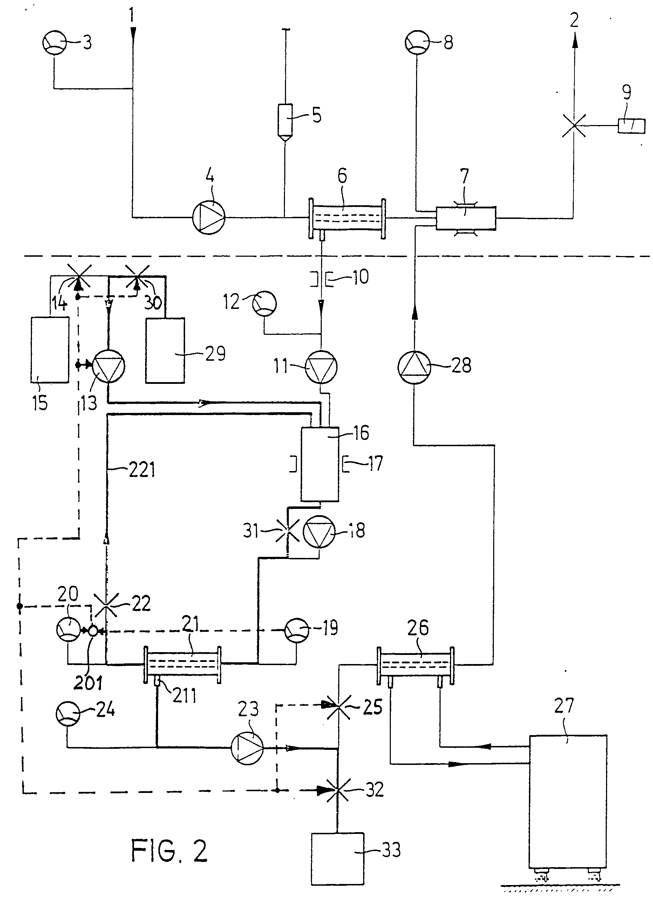

Da der Anteil an Präzipitat im Behälter 16 und im Filter 21 ständig ansteigt, ist es notwendig, in bestimmten Abständen das Präzipitat aus dem Behälter 16 und dem Rezirkulationskreislauf zu entfernen. Dies geschieht in der in Figur 2 dargestellten Betriebsweise der Vorrichtung. Zunächst wird der Plasmazufluß über die erste Pumpe 11 und der Zufluß von Acetatpuffer mit Heparin über die zweite Pumpe 13 gestoppt, indem diese Pumpen stillgesetzt werden. Auch die Pumpe 18 wird stillgesetzt und das Umgehungsventil 31 wird geöffnet. Eventuell ist es auch möglich, die Pumpe 18 weiterlaufen zu lassen. In diesem Fall kann das Umgehungsventil 31 entfallen. Die dritte Pumpe 23 saugt nun durch das Filter 21 hindurch Plasma an, das von den low-density-Lipoproteinen befreit ist und pumpt dieses Plasma durch das Dialysegerät 26 zur Pumpe 28. Wenn der Behälter 16 leergelaufen ist, erhöht sich die Druckdifferenz zwischen Einlaß und Auslaß des Filters 21. Diese Erhöhung der Druckdifferenz wird an einem Differenzbildner 201, der die Differenz zwischen den Signalen der Druckmesser 19 und 20 bildet, festgestellt. Wenn die Druckdifferenz einen bestimmten Schwellwert überschritten hat, läuft die Pumpe 13 an und das Ventil 32 öffnet und das Ventil 25; schließt. Außerdem schließt Ventil 14 und Ventil 30 öffnet. Nun wird aus dem Gefäß 29 dem Behälter 16 Lösungsmittel zugeführt. Das Ventil 22 öffent und die Rezirkulationspumpe 18 läuft an. Durch Rezirkulation des Lösungsmittels wird das in den Leitungen und im Filter 21 verbliebene Präzipitat gelöst. Nach Erreichen des Arbeitsspiegels im Behälter 16 läuft die dritte Pumpe 23 an und fördert das Lösungsmittel mit dem gelösten Präzipitat über das geöffnete Ventil 32 in den Behälter 33. Wenn sich das Präzipitat in ausreichendem Maße gelöst hat, wird an dem Differenzbildner 201 ein unterer Druckwert festgestellt. Darauhin wird die Pumpe 13 abgeschaltet und das Ventil 30 wird geschlossen. Derselbe Vorgang kann auch dadurch gelöst werden, daß der Druckmesser 24 einen relativ geringen Unterdruck anzeigt. Die dritte Pumpe 23 läuft noch solange, bis der Inhalt des Behälters 16 und des Kreislaufs in den Ablauf 33 gefördert ist. Danach wird ein neuer Zyklus zur Präzipitation der low-density-Lipoproteine gestartet.

## Patentansprüche

1. Vorrichtung zur extra-korporalen Präzipitation von Blutbestandteilen, mit einem Behälter, dem Blut oder Blutbestandteile eines Patienten über eine erste Pumpe zugeführt werden, und einem dem Behälter nachgeschalteten Filter dessen Filtrat-Auslaß mit einer zum Patienten zurückführenden Leitung verbunden ist, dadurch gekennzeichnet, daß das Filter (21) einen zweiten Auslaß für das Präzipitat aufweist, der mit einer in den Behälter (16) führenden Rücklaufleitung (221) verbunden ist, daß der Behälter (16) über eine zweite Pumpe (13) mit dem Auslaß eines ein Behandlungsmittel enthaltenden ersten Gefäßes (15) verbunden ist, und daß der Filtrat-Auslaß (211) des Filters (21) an eine dritte Pumpe (23) angeschlossen ist, deren Förderleistung im wesentlichen der Summe der Förderleistungen der ersten und der zweiten Pumpe entspricht.

2. Vorrichtung nach Anspruch 1, dadurch gekennzeichnet, daß die Förderleistung der ersten Pumpe (11) in Abhängigkeit von den Verhältnissen der Blutentnahme am Patienten veränderbar ist und daß die Antriebe der ersten Pumpe (11), der zweiten Pumpe (13) und der dritten Pumpe (23) miteinander derart gekoppelt sind, daß das Verhältnis der drei Förderleistungen konstant bleibt.

3. Vorrichtung nach Anspruch 2, dadurch gekennzeichnet, daß der Behälter (16) mindestens einen Füllstandsdetektor (17) aufweist, der bei einem oberen Füllstand die erste Pumpe (11) auf eine geringere Förderleistung umstellt oder abstellt.

4. Vorrichtung nach Anspruch 3, dadurch gekennzeichnet, daß eine den Strömungswiderstand des Filters (21) messende Einrichtung (19, 20, 201; 24) vorgesehen ist, die bei einem oberen Strömungswiderstand die zweite Pumpe (13) einschaltet, und aus einem ein Lösungsmittel enthaltenden zweiten Gefäß (29) Lösungsmittel in den Behälter (16) fördert und eine Verbindung zwischen dem Auslaß (211) des Filters (21) und dem Ablauf (33) herstellt.

## Revendications

1. Dispositif pour la précipitation extra-corporelle d'éléments constitutifs du sang, comportant un récipient, auquel du sang ou des éléments constitutifs du sang d'un patient sont envoyés par l'intermédiaire d'une première pompe, et un filtre, branché en aval du récipient et dont la sortie du filtrat est reliée à un conduit retournant au patient, caractérisé en ce que le filtre (21) possède une seconde sortie pour le précipité, qui est reliée à un conduit de retour (221) aboutissant au récipient (16), en ce que le récipient (16) est relié par l'intermédiaire d'une seconde pompe (13) à la sortie d'un premier récipient (15) contenant un agent de traitement, et en ce que la sortie du filtrat (211) du filtre (21) est raccordée à une troisième pompe (23), dont le débit de refoulement correspond essentiellement à la somme des débits de refoulement des première et seconde pompes.

2. Dispositif selon la revendication 1, caractérisé en ce que le débit de refoulement de la première pompe (11) peut varier en fonction des conditions du prélèvement du sang dans le patient, et en ce que les dispositifs d'entraînement de la première pompe (11), de la seconde pompe (13) et de la troisième pompe (23) sont accouplés entre eux de telle sorte que le rapport des trois débits d'entraînement reste constant.

3. Dispositif selon la revendication 2, caractérisé en ce que le récipient (16) comporte au moins un détecteur (17) du niveau de remplissage qui, pour un état de remplissage supérieur, commute la première pompe (11) sur un débit de refoulement plus faible ou l'arrête.

4. Dispositif selon la revendication 3, caractérisé en ce qu'il est prévu un dispositif (19, 20, 201; 24), qui mesure la résistance d'écoulement du filtre (21) et, pour une résistance d'écoulement supérieure, branche la seconde pompe (13), et entraîne un solvant à partir d'un second récipient (29) contenant ce solvant, en l'introduisant dans le récipient (16) et établit une liaison entre la sortie (211) du filtre (21) et l'évacuation (33).

**Claims**

1. An apparatus for the extracorporeal precipitation of blood components, said apparatus comprising a container to which blood or blood components of a patient are supplied through a first pump, and a filter downstream connected to said container, the filtrate outlet of said filter being connected to a conduit leading back to said patient, characterized in that the filter (21) has a second outlet for the precipitate, said outlet being connected to a return conduit (221) leading back to the container (16), that said container (16) is connected through a second pump (13) with the outlet of a first reservoir (15) containing a treating agent, and that the filtrate outlet (211) of said filter (21) is connected to a third pump (23), the volumetric output from which third pump essentially corresponding to the sum of the volumetric outputs of the first and second pumps.

2. The apparatus according to claim 1, characterized in that the volumetric output of the first pump (11) is variable as a function of the conditions of withdrawing blood from said patient and that the individual driving means of the first pump (11), the second pump (13) and the third pump (23) are coupled together in a manner such that the ratio of the three volumetric outputs remains constant.

3. The apparatus according to claim 2, characterized in that the container (16) has at least one fluid level detector means (17) which at an upper limit of the fluid level will cause the first pump (11) to be switched to a lower volumetric output or to be turned off.

4. The apparatus according to claim 3, characterized in that a device (19, 20, 201; 24) measuring the flow resistance of the filter (21) has been provided which device, at an upper limit of the flow resistance, turns on the second pump (13) which causes solvent to be discharged to the container (16) from a second reservoir (29) containing a solvent and which opens a connection between the outlet (211) of the filter (21) and the drain (33).

**EP 0 180 720 B1**

FIG. 1

FIG. 2